(19) Europäisches Patentamt
European Patent Office
Office européen des brevets

(11) Publication number: **0 402 033 B1**

# (12) EUROPEAN PATENT SPECIFICATION

(45) Date of publication of patent specification: **18.10.95**

(51) Int. Cl.[6]: **C07F 9/40**, A61K 31/66

(21) Application number: **90305873.3**

(22) Date of filing: **30.05.90**

The file contains technical information submitted after the application was filed and not included in this specification

## (54) Carboxamide derivatives.

(30) Priority: **30.05.89 JP 138685/89**
**01.05.90 JP 116525/90**

(43) Date of publication of application:
**12.12.90 Bulletin 90/50**

(45) Publication of the grant of the patent:
**18.10.95 Bulletin 95/42**

(84) Designated Contracting States:
**AT CH DE DK ES FR GB IT LI NL SE**

(56) References cited:
**EP-A- 0 273 444**
**US-A- 4 822 780**

**PAJP vol.10. no. 350 c 387 2406 November 26th 1986**

(73) Proprietor: **OTSUKA PHARMACEUTICAL FACTORY, INC.**
**115, Kuguhara, Tateiwa, Muyacho**
**Naruto-shi, Tokushima-ken (JP)**

(72) Inventor: **Tsutsumi, Kazuhiko**
**428-7, Tenjin,**
**Kamisuketocho**
**Tokushima-shi (JP)**
Inventor: **Uesaka, Eiji**
**169-1, Nakajima,**
**Narutocho Takashima**
**Naruto-shi (JP)**
Inventor: **Shinomiya, Kayoko**
**44 Nakamaegawacho-1-chome**
**Tokushima-shi (JP)**
Inventor: **Tsuda, Yoshihiko**
**127, Maehama**
**Muyacho Kokuwajima,**
**Naruto-shi (JP)**
Inventor: **Shoji, Yasuo**
**96-1, Mitsuho Kaitaku,**
**Mitsuho, Matsushigecho**
**Itano-gun,**
**Tokushima-ken (JP)**
Inventor: **Shima, Atsushi**
**22-6, Higashibiraki,**
**Nakamura,**
**Kitajimacho**
**Itano-gun,**
**Tokushima-ken (JP)**

(74) Representative: **Lamb, John Baxter et al**
**MARKS & CLERK,**
**57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to carboxamide derivatives. More particularly, the invention relates to said carboxamide derivatives, process for preparing the same, as well as a pharmaceutical composition for treating hyperlipidemia containing, as the active ingredient, said carboxamide derivative.

Because of recent improvements of living conditions and westernization of eating habits in these days, as well as increasing of the aging population, there has been reported rapid increasing of number of the patients who are suffered from hyperlipidemia and from arteriosclerosis caused thereby.

Generally, hyperlipidemia is defined as pathogenic conditions due to abnormal increasing of the serum lipids, thus due to abnormal increasing of any one of cholesterols triglycerides of phospholipids or free fatty acids contained in the blood. Furthermore, hyperlipidemia is considered as one of the important risk factors of arteriosclerosis, especially coronary arteriosclerosis, so that developments of methods for treating and preventing these diseases become social subject against the increasing of number of the patients who are suffered from such diseases.

As to methods for treating and curing hyperlipidemia, there have been applied several therapies such as dietetic therapy, therapeutic excercise and drug therapy and the like. A number of drugs have been studied and developed for the above-mentioned drug therapy, and some of them are now commercially available. Among such commercially available drugs, Clofibrate-type drugs, for example, have been developed for the purpose of lowering the concentration of lipids such as cholesterols, triglycerides and the like in the blood. These known drugs are classified in terms of their pharmacological mechanisms as follows:

(1) Lipid absorption inhibiting agent;
(2) Lipid biosynthesis inhibiting agent;
(3) Lipid dissimilation-excretion promoting agent;
(4) Lipoprotein metabolism improving agent; and
(5) Peroxidized lipid lowering agent.

It is important to the drugs for treating and curing hyperlipidemia that they are specifically required to have their safety as higher as possible, because these drugs are to be administered for long period due to the characters of the disease.

However, the drugs for treating and curing hyperlipidemia known in the art and thus are used widely in these days, for example Clofibrate shows certain drawbacks and side-effects such as they induce efflorescence, muscle ache, tenderness, liver function disturbance, etc., thus in using of these types of drugs, there should be paid considerable medical attentions and observations. According to a report on animal test results conducted in a foreign country, there was observed the formation of hepatic tumor when a large amount of Clofibrate was administered for a long period of time. [Cf. "YAKKYOKU" (Pharmacy), Vol. 31, No. 11, page 31, (1980)].

Other drugs for treating hyperlipidemia, other than the above-mentioned Clofibrate-type drugs, the safty thereof are not good enough, thus some of them induce hepatopathy as a side-effect which will cause important problems when they are administered for long period of time.

Furthermore, anyone of commercially available drugs for treating and curing hyperlipidemia cannot show lipid lowering activity, so that any drugs for use in these purpose having more excellent pharmacological activities have been expected to be developed for many years.

As to prior art literatures which may indicate carboxamide compounds similar to the carboxamide derivatives according to the present invention, there have been known Japanese Patent Application Kokai (Laid-open) No. 61-151199 (1986) and EP-A-273444, filed by the present applicants. However, these prior art literatures do not disclose at all the specific descriptions relating to the carboxamide derivatives according to the present invention. These prior art literatures disclose that carboxamide compounds are only useful as anti-inflammatory agents and calcium antagonizing agents, but the literatures do not teach anything about the usefulness of said carboxamide compounds as for treating and preventing of hyperlipidemia.

## SUMMARY OF THE INVENTION

An object of the present invention is to provide novel drugs for treating and curing hyperlipidemia, having excellent pharmacological activities, especially having the activity for lowering lipids in the blood with high safety, i.e., less side-effects such as lower toxicity, by eliminating all of these drawbacks shown by conventional known drugs for these purposes. Said features of the carboxamide derivatives according to the present invention could not have been expected and anticipated by carboxamide compounds known in prior

art literatures.

Another object of the present invention is to provide process for preparing the carboxide derivatives.

Further object of the present invention is to provide a pharmaceutical composition for treating hyperlipidemia containing, as the active ingredient, a specific carboxamide derivatives

DETAILED EXPLANATION OF THE INVENTION

In view of the above-mentioned circumstances of known drugs for treating hyperlipidemia, the present inventors have made extensive studies to develop the drugs, and found the fact that the specific carboxamide derivatives represented by the formula (1) as shown below possess pharmacological activities suitable for treating and curing of hyperlipidemia, especially they possess excellent activities in lowering lipids in the blood, as well as having lower toxicity with higher safety. Thus the present invention was successfully completed.

According to the present invention, carboxamide derivatives represented by the formula (1) is provided,

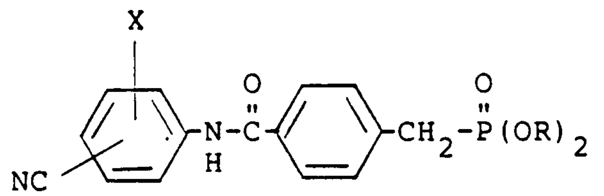

(1)

[wherein R is a lower alkyl group; X is a chlorine or bromine atom in the para-position and the group CN is in the ortho-position on the phenyl ring; or X is a bromine atom in the ortho-position and the group -CN is in the para-position].

In the general formula (1), as to the lower alkyl group indicated by the symbol R can be exemplified for example methyl, ethyl, propyl, isopropyl, butyl, <u>tert</u>-butyl, pentyl, hexyl groups and the like.

The carboxamide derivatives represented by the formula (1) possess excellent activities for lowering lipids in the blood and are useful agents for treating and curing hyperlipidemia, such as hyper-cholesterolemia, hyper-triglyceridemia, hyper-phospholipidemia, hyper-free fatty acidemia and the like.

The carboxamide derivatives represented by the formula (1), having excellent activities for lowering the concentration of lipids in the blood, and thus are useful agents for preventing and treating hyperlipidemia, have CN- group, as one of the substituents, at ortho- or para-position in the phenyl ring against the amino group, and a halogen atom represented by the symbol X is bonded at para- or ortho-position in the phenyl ring as indicated in the formula (1a) and (1b) as follows:

Formula (1a):

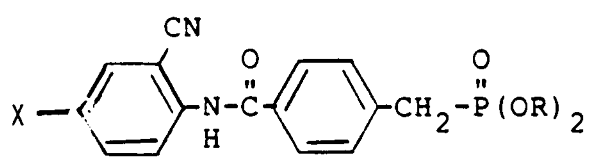

(1a)

[wherein R is the same as defined above and X is Br, Cl].

Formula (1b):

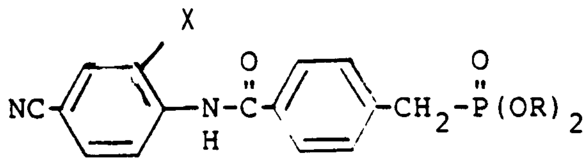

(1b)

[wherein R is the same as defined above and X is Br].

The carboxamide derivatives represented by the formula (1), have excellent activities for lowering the concentration of lipids in the blood, in addition to these activities, they also possess excellent safety without having side-effects such as hemolytic activity, thus the carboxamide derivatives represented by the formula (1) are quite useful agents for treating and preventing the above-mentioned diseases.

The carboxamide derivatives represented by the formula (1) of the present invention can be prepared by various methods, and a typical process can be illustrated by reaction scheme-1 as follows:

Reaction scheme-1

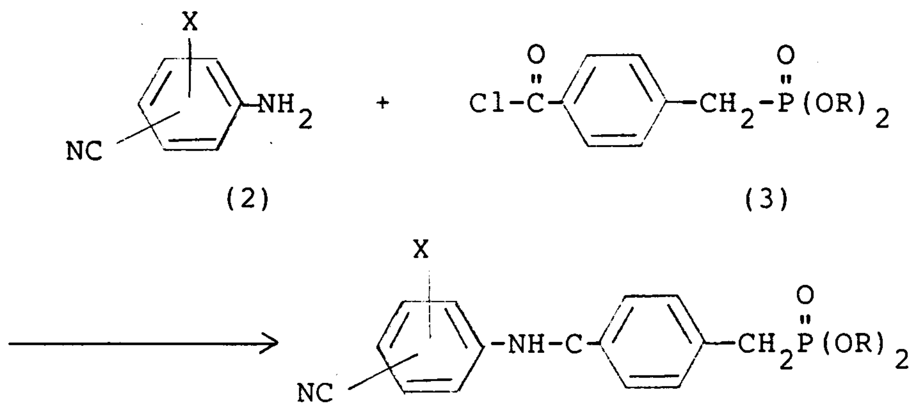

(2) + (3)

(1)

[wherein R and X are each the same as defined above].

According to the reaction scheme-1, a carboxamide derivative (1) of the present invention can be prepared by reacting an amine (2) with a carboxylic acid chloride derivative (3).

The reaction is carried out, generally in a suitable solvent in the presence of a deacidifying agent. As to the deacidifying agent used in the reaction, any type of deacidifying agent used to this purpose which does not give any adverse effect can be employed. As to the examples thereof, a tertiary amine such as triethylamine, N,N-diethylaniline, N-methylmorpholine, pyridine, 4-dimethylaminopyridine and the like can preferably be exemplified. As to the solvent used to the reaction, aromatic and aliphatic hydrocarbons such as benzene, toluene, xylene, petroleum ether and the like; chain and cyclic ethers such as diethyl ether, dimethoxyethane, tetrahydrofuran (THF), 1,4-dioxane and the like; ketones such as acetone, methyl ethyl ketone, acetophenone and the like; and halogenated hydrocarbons such as dichloromethane, chloroform, carbon tetrachloride, 1,2-dichloroethane and the like can be exemplified.

The ratio of the amount of a carboxylic acid chloride (3) to the amount of an amine (2) in the above-mentioned reaction is specifically restricted, and generally an equimolar quantity to an excess quantity of the former may be used to the latter. The above-mentioned deacidifying agent may preferably be used, generally in an equimolar to samall excess quantity to the carboxylic acid chloride (3). The reaction can be proceeded under any condition such as under cooling, at room temperature or under heating, and generally the reaction may be carried out under temperature condition within the range of at room temperature to the refluxing temperature of a solvent to be used, and is completed generally within about 0.5 to 10 hours.

The objective carboxamide derivative (1) of the present invention obtained by the above-mentioned reaction scheme-1 can be isolated from the reaction system by usual separation means, and then can be purified. As to the above-mentioned isolation and separation means, conventional solvent extraction,

distillation, recrystallization, column chromatography, preparative thin layer chromatography and the like can be employed.

The objective carboxamide derivatives (1) of the present invention can be used in the form of any type of conventional preparations of pharmaceutical composition. The above-mentioned preparations of pharmaceutical composition are prepared by using conventional diluents such as fillers, bulking agents, binders, wetting agents, disintegrating agents, surface active agents, lubricants and the like or excipients. As to the type of preparations of pharmaceutical composition, it can be selected depend on various therapeutical purposes, for example tablets, pills, powders, liquids, suspensions, emulsions, granules, capsules, suppositories, injections (liquids, suspensions, etc.) and the like can be exemplified. In case of preparing the pharmaceutical compositions in the form of tablets, there can be prepared by using excipients such as lactose, white sugar, sodium chloride, glucose, urea, starch, calcium carbonate, kaolin, crystalline cellulose, silicic acid and the like; binding agents such as water, ethanol, propanol, simple syrup, glucose solutions, starch solutions, gelatin solutions, cabroxymethyl cellulose, shelac, methylcellulose, potassium phosphate, polyvinylpyrrolidone and the like; disintegrating agets such as carboxylmethyl cellulose or its calcium salt, microcrystalline cellulose, sodium alginate, agar-agar powder, laminaran powder, sodium hydrogen carbonate, calcium carbonate, fatty acid esters of polyoxyethylene solbitol, sodium laurylsulfate, monoglyceride of stearic acid, starch, lactose and the like; disintegration inhibitors such as white sugar, stearin, coconut butter, hydrogenated vegetable oils and the like; absorption accelarating agents such as quaternary ammonium bases, sodium laurylsulfate and the like; wetting agents such as glycerin, starch and the like; absorbing agents such as starch, lactose, kaolin, bentonite, colloidal silicic acid and the like; lubricants such as purified talc, stearates boric acid powder, polyethylene glycols and the like, can be exemplified. If necessary, the tablets can be further coated with usual coating materials to make them into coated tablets, for example tablets coated with sugar, tablets coated with gelatin film, tbalets coated with enteric coating layers, tablets coated with films or double layered tablets as well as multiple layered tablets and the like.

In case of preparing the pharmaceutical compositions in the form of pills, any carrier which is known and used widely in this field can be used, for example excipients such as glucose, lactose, starch, coconut butter, hydrogenated vegetable oils, kaolin, talc and the like; binding agents such as powdered gum arabi, powdered tragacanth gum, gelatin, ethanol and the like; disintegrating agents such as laminaria, agar-agar and the like.

In case of preparing the pharmaceutical compositions in the form of suppositories, any carrier which is known and used widely in this field can be used, for example polyethylene glycols, coconut butter, higher alcohols, esters of higher alcohols, gelatin, semi-synthesized glycerides and the like.

In case of preparing the pharmaceutical compositions in the form of capsules, the carboxamide derivative (1) of the present invention can be admixed with the above-mentioned carriers being exemplified, and thus obtained mixture is filled in a solid gelatin capsule or in a soft capsule.

In case of preparing the pharmaceutical compositions in the form of injection preparations, solutions, emulsions or suspensions are further sterilized and they are preferably isotonic to the blood. In preparing the injection preparations, any carriers which are known widely and used in this field can be employed, for example water, ethanol, polyethylene glycol, propylene glycols, ethoxylated isostearyl alcohol, polyoxylated isostearyl alcohol, fatty acid esters of polyoxyethylene solbitol and the like. In these instances, adequate amounts of sodium chloride, glucose or glycerin may be added to the objective injection preparations to make them isotonic to the blood. Furthermore, usual dissolving agents, buffer solutions, analgesic agents and the like may be added thereto. Also , if necessary, coloring agents, preservatives, perfumes, seasoning agents, sweetening agents and other medicines may be added into the objective pharmaceutical composition preparations.

The amount of the carboxamide derivative (1) of the present invention to be contained in the pharmaceutical composition according to the present invention is not specifically restricted, and can be suitbaly selected from a wide range, and generally 1 to 70% by weight of the carboxamide derivative (1) may be contained in the pharmaceutical composition.

Methods for administering the above-mentioned pharmaceutical compositions are not specifically restricted, and they can be determined depending on the type of each of the pharmaceutical compositions, the age, the distinction of sex, the degree of symptoms and other conditions of the patient. For example, tablets, pills, liquids, suspensions, emulsions, granules and capsules are administered orally. Injection preparations are administered intravenously singly or administered with usual injectable transfusions such as glucose solutions, amino acid solutions and the like, and if necessary, the injection preparations are administered singly intramuscularly, intracutaneously, subcutaneously or intraperitoneally. Suppositories are administered into the rectum.

Administration dosage of the above-mentioned pharmaceutical compositions can be suitably selected depend on the methods of administration, the age of the patient, the distinction of sex and other conditions, as well as the degree of the symptoms, and generally the pharmaceutical compositions may contain about 0.05 to 80 mg of carboxamide derivative (1) per kg of the body weight per day and the pharmaceutical compositions may be administered dividedly 1 to 4 times a day.

EXAMPLES

In order to explain the present invention more in detail, examples of preparation of the carboxamide derivatives (1), pharmacological tests and examples of preparations of pharmaceutical compositions are illustrated as follows.

Example 1

Preparation of 4-diethoxyphosphinoylmethyl-N-(4-bromo-2-cyanophenyl)benzamide

3.94 Grams (20 mM) of 2-amino-5-bromobenzonitrile, 2.22 g (22 mM) of triethylamine and 0.49 g (4 mM) of 4-dimethylaminopyridine were dissolved in 40 ml of dried dichloromethane. To this solution was added dropwise slowly 40 ml of dried dichloromethane solution containing 5.81 g (20 mM) of 4-diethoxyphosphinolymethylbenzoyl chloride, with stirring under ice-cooling condition.
After the stirring was continued for 10 hours at room temperature, 50 ml of water was added to the reaction mixture, and the whole mixture was extracted with chloroform. The chloroform extract was dried over anhydrous sodium sulfate and the solvent was removed by evaporation under reduced pressure. The obtained residue was purified by means of a silica gel column chromatography (eluted with a mixture of chloroform : ethyl acetate = 1 : 2), then recrystallized from benzene-n-hexane to yield 2.94 g of 4-diethoxyphosphinoylmethyl-N-(4-bromo-2-cyanophenyl)benzamide as colorless crystals. Melting point: 165-166 °C. (Recrystallized from benzene-n-hexane).

Examples 2-5

By procedures similar to those employed in Example 1, there were prepared carboxamide compounds as shown in Table 1 as follows. In Table 1, the chemical structural formula of carboxamide compound prepared in Example 1 is also indicated.

Table 1

| Example No. | Chemical structural formula | Melting point (°C) (Recrystallization solvent) |
|---|---|---|
| 1 | 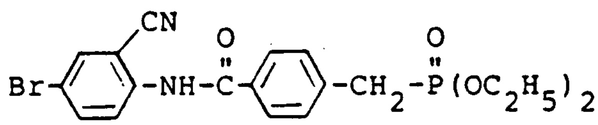 | 165-166 (Benzene-n-hexane) |
| 2 | 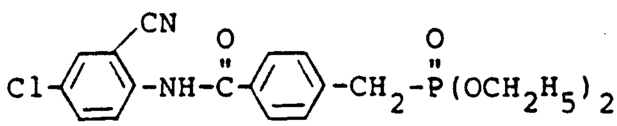 | 171-172 (Chloroform-n-hexane) |
| 3 | 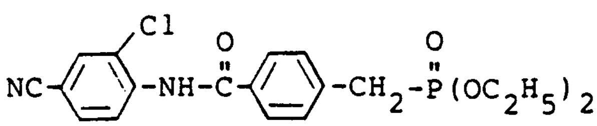 | 152-154 (Benzene-n-hexane) |
| 4 | 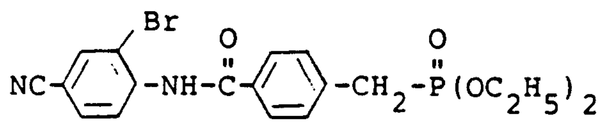 | 152-154 (Benzene-n-hexane) |
| 5 | 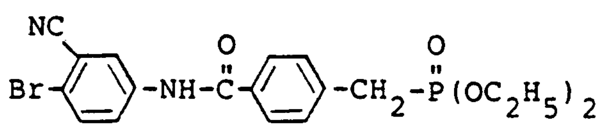 | 208.5-209.5 (Chloroform-n-hexane) |

Pharmacological Test - 1

Seven-week-old Wistar strain male rats were used as the test animals. One test group consisting of six (6) rats. A test compound was suspended in a 0.5%-carboxymethyl cellulose (CMC) aqueous solution, thus prepared suspension containing the test compound was orally administered compulsorily by using a sonde to the rat in the amount of 300 mg/5 ml/kg/day for 2 days. Similar to the test group, to each of six (6) rats in the control group was administered with 0.5%-CMC aqueous solution without containing the test compound.

After the final administration was finished, the test rat was fasted for 20 hours, and the blood sample was taken from the rat. The concentration of plasma high density lipoprotein cholesterol (HDLC) was determined by using a measuring instrument of HDL-C kit-N (manufactured by Nihon Shoji Kabushiki Kaisha).

Increasing ratio (%) of plasma HDLC concentration performed by a test compound was calculated from the formula as follows:

$$\text{Increasing ratio (\%)} = \frac{\text{[Plasma HDLC concentration of test group]}}{\text{[Plasma HDLC concentration of control group]}} \times 100$$

The results obtained are shown in Table 2 as follows.

Table 2

| Test compound | Increasing ratio (%) of the plasma HDLC concentration |
|---|---|
| Compound of Example 1 | 193 |
| Compound of Example 2 | 196 |
| Compound of Example 3 | 187 |
| Compound of Example 4 | 222 |

Pharmacological Test - 2 (Hemolysis test)

Seven-week-old ddY-strain male mice were used as test animals. One test group consisting of six (6) mice. A test compound was suspended in a 0.5%-carboxymethyl cellulose (CMC) aqueous solution, thus prepared suspension containing the test compound was orally administered compulsorily by using a sonde to the mouse in the amount of 600 mg/5 ml/kg/day for 10 days. Similar to the above, to each of six mice in the control group was administered with 0.5%-CMC aqueous solution without containing the test compound.

After the final administration was finished, the mouse was fasted for 20 hours, then the mouse was subjected to dissection and the blood sample as well as the sample of the spleen were taken by enucleation. The number of the erythrocytes was counted and the weight of the spleen was measured.

The ratio of number of the erythrocytes was calculated by dividing the mean value of number of the erythrocytes obtained from the test group by the mean value of number of the erythrocytes obtained from the control group. Similarly, the ratio of weight of the spleen was calculated by dividing the mean value of weight of the spleen obtained from the test group by the mean value of weight of the spleen obtained from the control group. The results are shown in Table 3 as follows.

Table 3

| Test compound | Ratio of number of the erythrocytes | Ratio of weight of the spleen |
|---|---|---|
| Compound of Example 1 | 0.96 | 1.13 |
| Compound of Example 2 | 0.97 | 1.09 |
| Compound of Example 4 | 1.07 | 0.94 |

As can be seen from the data shown in Table 3, the carboxamide derivatives of the present invention do not have hemolytic activity as side-effect, because any significant differences are not observed between the data obtained from the test groups and the data obtained from control groups.

Example of Preparation of Pharmaceutical Composition - 1

Preparation of Tablets

Tablets (1,000 tablets) each of which containing 250 mg of 4-diethoxyphosphinoylmethyl-N-(4-bromo-2-cyanophenyl)benzamide (hereinafter referred to as "Compound A") as the active ingredient were prepared with the following formulation.

| Ingredients | Amount (g) |
|---|---|
| Compound A | 250 |
| Lactose (Japanese Pharmacopoeia grade) | 33.3 |
| Corn starch (Japanese Pharmacopoeia grade) | 16.4 |
| Calcium carboxymethyl cellulose (Japanese Pharmacopoeia grade) | 12.8 |
| Methyl cellulose (Japanese Pharmacopoeia grade) | 6.0 |
| Magnesium stearate (Japanese Pharmacopoeis grade) | 1.5 |
| Total amount | 320.0 |

In accordance with the above-mentioned formulation, Compound A, lactose, corn starch and calcium carboxymethyl cellulose were thoroughly admixed together, then the mixture was shaped into granular form by using an aqueous solution of methyl cellulose, then thus obtained granules were passed through a sieve (# 24), the granules being passed through the sieve were mixed with magnesium stearate and then pressed into tablets form.

Example of Preparation of Pharmaceutical Composition - 2

Preparation of Capsules

Hard gelatin capsules (1,000 capsules) each of which containing 4-diethoxyphosphinoylmethyl-N-(2-chloro-4-cyanophenyl)benzamide (hereinafter referred to as "Compound B") as the active ingredient were prepared with the following formulation.

| Ingredients | Amount (g) |
|---|---|
| Compound B | 250 |
| Crystalline cellulose (Japanese Pharmacopoeia grade) | 30 |
| Corn starch (Japanese Pharmacopoeia grade) | 17 |
| Talc (Japanese Pharmacopoeia grade) | 2 |
| Magnesium stearate (Japanese Pharmacopoeia grade) | 1 |
| Total amount | 300 g |

In accordance with the above-mentioned formulation, each of the ingredients was finely pulverized, then they were admixed thoroughly so as to form a uniform mixture. The mixture was filled in a gelatin capsules for oral administration, having the desired size, so as to prepare the objective capsule preparation.

Example of Preparation of Pharmaceutical Composition - 3

Preparation of Granules

Granules (1,000 g), 1 g each of which containing 500 mg of 4-diethoxyphosphinoylmethyl-N-(2-bromo-4-cyanophenyl)benzamide (hereinafter referred to as "Compound C") as the active ingredient were prepared with the following formulation.

| Ingredients | Amount (g) |
|---|---|
| Compound C | 500 |
| Corn starch (Japanese Pharmacopoeia grade) | 250 |
| Lactose (Japanese Pharmacopoeia grade) | 100 |
| Crystalline cellulose (Japanese Pharmacopoeia grade) | 100 |
| Calcium carboxymethyl cellulose (Japanese Pharmacopoeia grade) | 40 |
| Hydroxypropyl cellulose (Japanese Pharmacopoeia grade) | 10 |
| Total amount | 1,000 g |

In accordance with the above-mentioned formulation, Compound C, corn starch, lactose, crystalline cellulose and calcium carboxymethyl cellulose were admixed thoroughly, then an aqueous solution of hydroxypropyl cellulose was added to the mixture and the whole mixture was kneaded, then by using a extruding-granulating machine to prepare granules, next the granules were dried at 50 °C for 2 hours to prepare the objective granular preparation.

## Claims

1. Carboxamide derivatives of the formula:

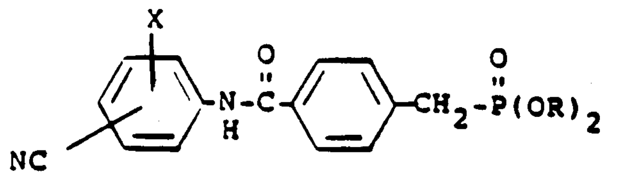

(1)

(wherein R is a lower alkyl group; X is a chlorine or bromine atom in the para-position and the group CN is in the ortho-position on the phenyl ring; or X is a bromine atom in the ortho-position and the group -CN is in the para-position).

2. A carboxamide derivative according to Claim 1 which is 4-diethoxyphosphinoylmethyl-N-(4-bromo-2-cyanophenyl) benzamide, 4-diethoxyphosphinoylmethyl-N-(4-chloro-2-cyanophenyl)benzamide, or 4-diethoxyphosphinoylmethyl-N-(2-bromo-4-cyanophenyl)benzamide,

3. A pharmaceutical composition for treating and curing hyperlipidemia characterized by containing, as active ingredient, a carboxamide derivative as claimed in Claim 1.

4. A pharmaceutical composition according to Claim 3 containing, as active ingredients, one or more of: 4-diethoxyphosphinoylmethyl-N-(4-bromo-2-cyanophenyl) benzamide and 4-diethoxyphosphinoylmethyl-N-(4-chloro-2 cyanophenyl)benzamide.

## Patentansprüche

1. Carbonsäureamid-Derivat der Formel:

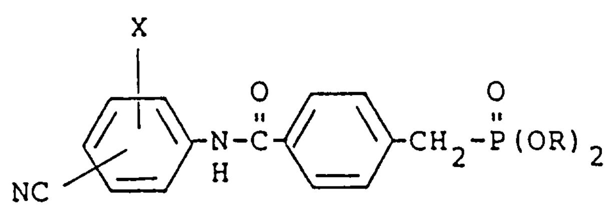

(1)

(worin R eine niedere Alkyl-Gruppe ist; X ein Chlor- oder Bromatom in der para-Stellung ist und die CN-Gruppe sich in ortho-Stellung am Phenylring befindet; oder X ein Bromatom in der ortho-Stellung ist und die CN-Gruppe sich in para-Stellung befindet).

2. Carbonsäureamid-Derivat gemäß Anspruch 1, welches 4-Diethoxyphosphinoylmethyl-N-(4-brom-2-cyanophenyl)benzamid, 4-Diethoxyphosphinoylmethyl-N-(4-chlor-2-cyanophenyl)benzamid oder 4-Diethoxyphosphinoylmethyl-N-(2-brom-4-cyanophenyl)benzamid ist.

3. Pharmazeutische Zusammensetzung zur Behandlung und Heilung von Hyperlipämie,
dadurch **gekennzeichnet**, daß
sie als wirksamen Bestandteil ein Carbonsäureamid-Derivat gemäß Anspruch 1 enthält.

4. Pharmazeutische Zusammensetzung gemäß Anspruch 3, die als wirksamen Bestandteil eine oder mehrere der Verbindungen:
4-Diethoxyphosphinoylmethyl-N-(4-brom-2-cyanophenyl)benzamid und
4-Diethoxyphosphinoylmethyl-N-(4-chlor-2-cyanophenyl)benzamid
enthält.

**Revendications**

1. Dérivés de carboxamide de formule :

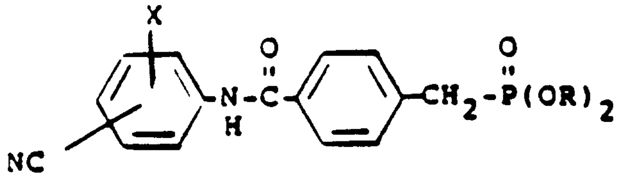

(1)

(dans laquelle R est un groupe alkyle inférieur ; X est un atome de chlore ou de brome dans la position para et le groupe -CN est dans la position ortho sur le cycle phényle ; ou X est un atome de brome dans la position ortho et le groupe -CN est dans la position para).

2. Dérivé de carboxamide selon la revendication 1, qui est le 4-diéthoxyphosphinoylméthyl-N-(4-bromo-2-cyanophényl)benzamide, le 4-diéthoxyphosphinoylméthyl-N-(4-chloro-2-cyanophénylbenzamide ou le 4-diéthoxyphosphinoylméthyl-N-(2-bromo-4-cyanophényl)benzamide.

3. Composition pharmaceutique pour le traitement et la guérison de l'hyperlipidémie, caractérisée en ce qu'elle contient, en tant qu'ingrédient actif, un dérivé de carboxamide selon la revendication 1.

4. Composition pharmaceutique selon la revendication 3, contenant comme ingrédients actifs un ou plusieurs du
4-diéthoxyphosphinoylméthyl-N-(4-bromo-2-cyanophényl)benzamide et du
4-diéthoxyphosphinoylméthyl-N-(4-chloro-2-cyanophényl)benzamide.